# EUROPEAN PATENT SPECIFICATION

(11) **EP 3 949 887 B1**
(45) Date of publication and mention of the grant of the patent: **07.05.2025**
(21) Application number: 20783586.9
(22) Date of filing: 25.03.2020
(51) Int. Cl.: A61B 18/24, G01M 11/00

(54) **OPTICAL FIBER STATE DETECTION SYSTEM**
GLASFASERZUSTANDSERKENNUNGSSYSTEM
SYSTÈME DE DÉTECTION D'ÉTAT DE FIBRE OPTIQUE

(30) Priority: 29.03.2019 JP 2019065799
(43) Date of publication of application: 09.02.2022
(73) Proprietor: Furukawa Electric Co., Ltd., Tokyo 100-8322 (JP)
(72) Inventor: NOMURA, Yoshiki, Tokyo 100-8322 (JP); WATANABE, Kengo, Tokyo 100-8322 (JP); MATSUSHITA, Shunichi, Tokyo 100-8322 (JP)
(74) Representative: SSM Sandmair
(86) International application number: PCT/JP2020/013355
(87) International publication number: WO 2020/203559

(56) References cited:
- EP-A1- 3 804 652
- WO-A1-2021/167085
- JP-A- 2011 154 001
- JP-A- 2017 213 444
- JP-A- 2018 534 110
- JP-A- 2019 023 580
- US-A1- 2008 285 017
- US-A1- 2015 141 969
- US-A1- 2017 079 718
- US-A1- 2017 354 465

## Description

### Field

The present invention relates to an optical fiber state detection system.

### Background

There is a known technique for providing a medical treatment by inserting a catheter with an optical fiber inserted therein into the patient's body. In such an optical fiber probe for medical use, since the diameter of the optical fiber becomes smaller with the decrease in the diameter of the catheter, there is a risk that the optical fiber will be strongly bent or broken when the catheter is inserted into the patient's body.

For example, Patent Literatures 1 and 2 disclose a technique for detecting the curvature of a tubular member, such as an endoscope, which is inserted into the body, or estimating the curved shape of the tubular member.

### Citation List

### Patent Literature

Patent Literature 1: Japanese Laid-open Patent Publication No. 2001-169998
Patent Literature 2: Japanese Laid-open Patent Publication No. 2015-181643

Further relevant documents are: US 2017/079718 A1, US 2008/285017 A1 and US 2015/141969 A1.

### Summary

### Technical Problem

When the optical fiber is strongly bent during insertion of the catheter, light will leak out at the curved portion. Therefore, for example, when an ablation medical treatment is performed with laser light, the optical power reaching a desired position could be reduced, or the ablation could be applied to a position not in need due to the leakage of light at the curved portion.

For the reasons describe above, there has been a need for a technique that accurately measures the bending loss of the optical fiber during insertion of the catheter, that is, when the optical fiber is in operation, and determines the presence or absence of a bend on the optical fiber. However, Patent Literatures 1 and 2 mentioned above do not disclose such a technique.

The present invention has been made in consideration of the above, and has an object to provide an optical fiber state detection system, which can accurately measure the bending loss of an optical fiber in operation, and determine the presence or absence of a bend on the optical fiber.

### Solution to Problem

The invention is defined in the appended claims. To resolve the problem and attain the object, an optical fiber state detection system according to an embodiment of the present invention includes: a first light source that outputs a monitor-related light for monitoring a state of an optical fiber; a reflection mechanism that reflects the monitor-related light propagated through the optical fiber; a light receiving part that receives a reflected light reflected by the reflection mechanism; a tap coupler provided between the reflection mechanism and both the first light source and the light receiving part such that the first light source and the light receiving part are connected the tap coupler; and a control part. Further, when the control part detects that a received optical power of the reflected light is greater than 0 and lower than a predetermined threshold value, the control part outputs information on a decrease in the received optical power to outside.

An optical fiber state detection system includes: a first light source that outputs a monitor-related light for monitoring a state of an optical fiber; a reflection mechanism that reflects the monitor-related light propagated through the optical fiber; a light receiving part that receives a reflected light reflected by the reflection mechanism; a tap coupler provided between the reflection mechanism and both the first light source and the light receiving part such that the first light source and the light receiving part are connected the tap coupler; a second light source that outputs an ablation-related light; a wave multiplexer that multiplexes the monitor-related light and the ablation-related light; and a control part. Further, the first light source and the second light source are connected to the wave multiplexer, the wave multiplexer and the light receiving part are connected to the tap coupler, the monitor-related light and the ablation-related light are different from each other in wavelength, the reflection mechanism transmits the ablation-related light, and when the control part detects that a received optical power of the reflected light is greater than 0 and lower than a predetermined threshold value, the control part shuts down the second light source.

In the optical fiber state detection system according to an embodiment of the present invention, a wavelength of the monitor-related light is a wavelength in a visible light wavelength bandwidth.

In the optical fiber state detection system according to an embodiment of the present invention, the monitor-related light is a flat top beam.

The monitor-related light and the ablation-related light are same as each other in beam shape.

In the optical fiber state detection system according to an embodiment of the present invention, a mechanism that cuts a wavelength of the ablation-related light is provided on a front side of the light receiving part.

### Advantageous Effects of Invention

According to the present invention, it is possible to accurately measure the bending loss of an optical fiber in operation, and determine the presence or absence of a bend on the optical fiber.

### Brief Description of Drawings

FIG. 1 is a schematic diagram illustrating a configuration example of an optical fiber state detection system according to a first embodiment of the present invention.
FIG. 2 is a diagram for explaining an example of the bending loss characteristic of an optical fiber, which is a graph showing the relationship between the wavelength of light and the bending loss of the optical fiber.
FIG. 3 is a schematic diagram illustrating a first configuration example for making a monitor-related light into a flat top beam in the optical fiber state detection system according to the first embodiment of the present invention.
FIG. 4 is a schematic diagram illustrating a second configuration example for making a monitor-related light into a flat top beam in the optical fiber state detection system according to the first embodiment of the present invention.
FIG. 5 is a diagram for explaining an example of the bending loss characteristic of an optical fiber depending on the core diameter of the optical fiber, which is a graph showing the relationship between the bending radius of the optical fiber and the bending loss.
FIG. 6 is a diagram illustrating a system configuration example according to an example 1 of FIG. 5.
FIG. 7 is a diagram illustrating a system configuration example according to an example 2 of FIG. 5.
FIG. 8 is a schematic diagram illustrating a configuration example of an optical fiber state detection system according to a second embodiment of the present invention.
FIG. 9 is a schematic diagram illustrating a configuration example for making a monitor-related light and an ablation-related light into the same beam shape in the optical fiber state detection system according to the second embodiment of the present invention.
FIG. 10 is a schematic diagram illustrating a first configuration example of an optical fiber state detection system according to a third embodiment of the present invention.
FIG. 11 is a schematic diagram illustrating a second configuration example of an optical fiber state detection system according to the third embodiment of the present invention.
FIG. 12 is a schematic diagram illustrating a third configuration example of an optical fiber state detection system according to the third embodiment of the present invention.

### Description of Embodiments

An optical fiber state detection system according to the present invention will be explained below with reference to the accompanying drawings. Note that the present invention is not limited to the following embodiments. Further, the constituent elements in the following embodiments encompass those which can be easily used as replacements by a person skilled in the art, or which are substantially equivalent thereto.

### (First Embodiment)

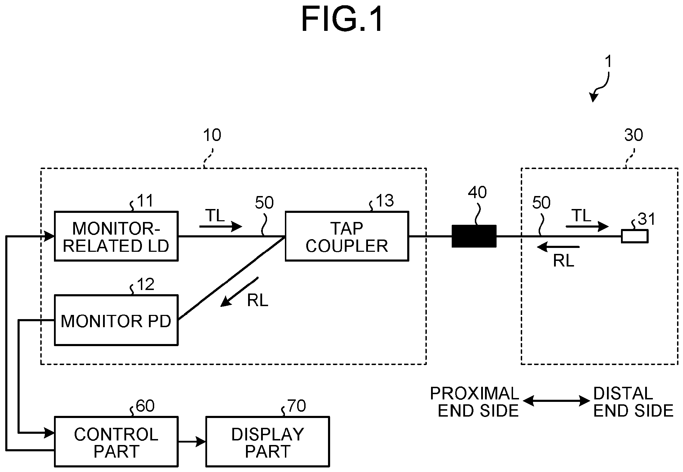

An optical fiber state detection system (which will be simply referred to as "state detection system", hereinafter) 1 according to this embodiment includes, as illustrated in FIG. 1, a laser apparatus 10, an optical fiber probe 30 equipped with a reflection mechanism 31, a connector 40 that connects the laser apparatus 10 and the optical fiber probe 30 to each other, optical fibers 50, a control part 60, and a display part 70. Here, the connector 40 is not essential, and thus may be omitted. Further, in FIG. 1, the optical fibers 50 are illustrated by solid lines.

The laser apparatus 10 includes a monitor-related LD 11, a monitor PD 12, a tap coupler 13, and optical fibers 50 that connect these components. Here, the "LD" stands for "laser diode", and the "PD" stands for "photodiode".

The monitor-related LD 11 serves as a light source (first light source) that outputs a monitor-related light TL for monitoring the state of an optical fiber 50. The monitor-related LD 11 is connected to the input side of the tap coupler 13 via an optical fiber 50.

The laser apparatus 10 may be provided with a plurality of monitor-related LDs 11 that output lights different from each other in wavelength. Alternatively, the monitor-related LD 11 may be formed of a structure integrally combing a plurality of LDs that output lights different from each other in wavelength. Here, the output of the monitor-related LD 11 is set to 1 mW or less, for example. Further, the wavelength of the monitor-related light TL is set to be a wavelength that falls within a range of the visible light wavelength bandwidth to the near infrared wavelength bandwidth (400 nm to 1,500 nm), and preferably to be a wavelength that falls within a range of the visible light wavelength bandwidth (400 nm to 700 nm).

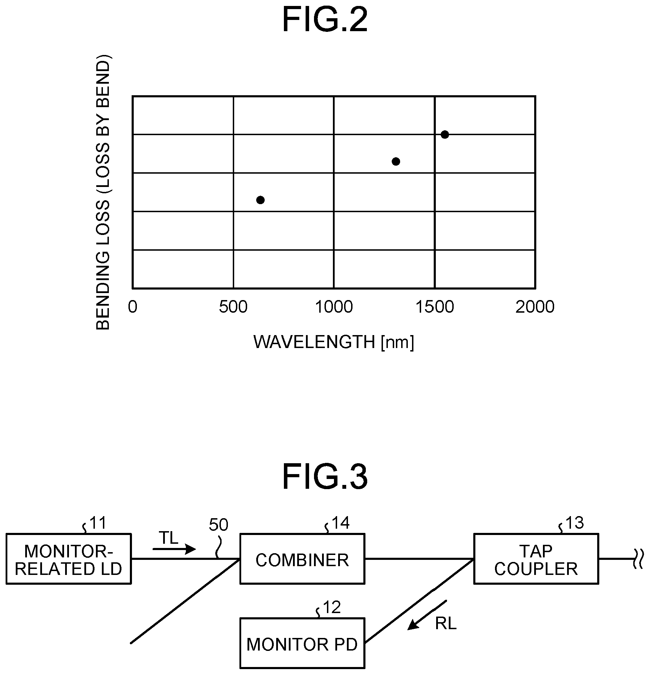

Now, FIG. 2 is a diagram for explaining an example of the bending loss characteristic of an optical fiber 50, which is a graph showing the relationship between the wavelength of light and the bending loss of the optical fiber 50. Here, the bending loss (loss by a bend) is defined by the amount of increase in transmission loss when the optical fiber 50 is bent with a predetermined bending radius, for example.

As illustrated in FIG. 2, the bending loss of the optical fiber 50 is smaller on the shorter wavelength side and larger on the longer wavelength side. This means that, for example, when the bending loss of the optical fiber 50 is measured by using a monitor-related light TL with a shorter wavelength (in the visible light wavelength bandwidth) and is determined that "there is a bend", it will be determined that "there is a bend" even if a monitor-related light TL with a longer wavelength (in a wavelength bandwidth longer than the visible light wavelength bandwidth) is used.

Therefore, as described above, when a light in the visible light wavelength bandwidth is used as the monitor-related light TL, it becomes possible to also determine the presence or absence of a bend on the optical fiber 50 of the case where a monitor-related light TL in a longer wavelength bandwidth is used. Here, for example, in a case where it is determined that "there is no bend" when a monitor-related light in the visible light wavelength bandwidth is used, and it is determined that "there is a bend" when a monitor-related light in a longer wavelength bandwidth is used, it may be acceptable to overestimate the bending loss by using a light in a wavelength bandwidth longer than the visible light wavelength bandwidth as the monitor-related light TL.

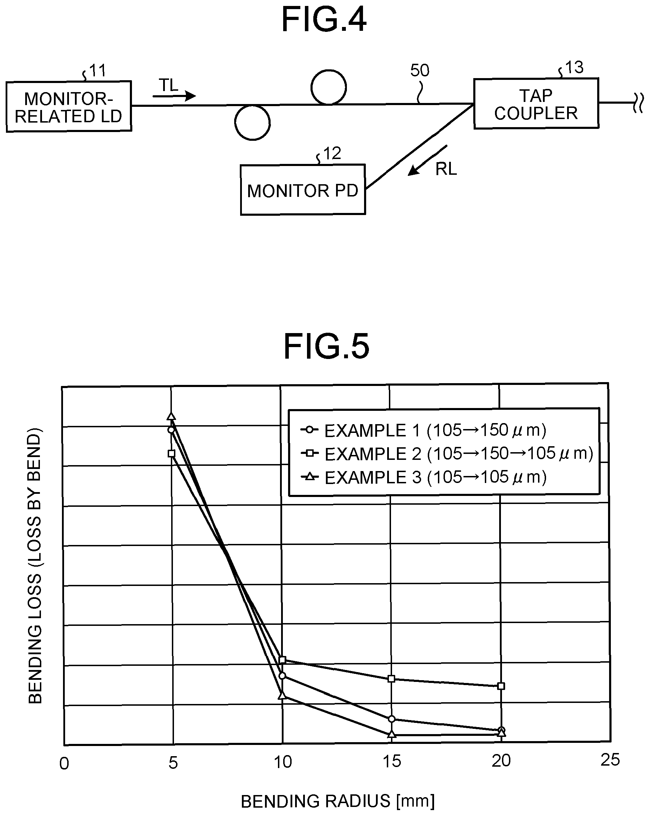

The monitor-related light TL is composed of a flat top beam (top hat beam). In order to make the monitor-related light TL into a flat top beam, for example, there is a method to be used, such as a method of arranging a combiner 14 between the monitor-related LD 11 and the tap coupler 13 as illustrated in FIG. 3, a method of performing mode mixing by applying a bend to the optical fiber 50 as illustrated by a circle in FIG. 4, or a method of applying vibration to the optical fiber 50.

Note that, in FIGS. 3 and 4, the optical fiber probe 30, the connector 40, the control part 60, and the display part 70 are omitted from the illustration. Here, as regards the combiner 14 illustrated in FIG. 3, the number thereof is not particularly limited, but may be set to two or more. Further, the bending radius of the optical fiber 50 illustrated in FIG. 4 is set to a value (for example r = 10 mm) of a long term bending radius (Long Term Bend Radius: LTBR) of the optical fiber 50, or the like. Returning to FIG. 1, an explanation will be given of the rest of the configuration.

The monitor PD 12 serves as a light receiving part that receives and monitors the reflected light RL reflected by the reflection mechanism 31. The monitor PD 12 is connected to the input side of the tap coupler 13 via an optical fiber 50. Here, a plurality of monitor PDs 12 may be arranged, as in the monitor-related LD 11, such that, for example, the plurality of monitor PDs 12 may receive lights different from each other in wavelength output from the plurality of monitor-related LDs 11.

The tap coupler 13 is arranged between the monitor-related LD 11 and monitor PD 12 and the reflection mechanism 31. The monitor-related LD 11 and the monitor PD 12 are connected to the input side of the tap coupler 13 via optical fibers 50. Further, the connector 40 is connected to the output side of the tap coupler 13. The tap coupler 13 is preferably an asymmetric tap coupler, and the composition ratio of the tap coupler 13 can be set to, for example, 99:1, 95:5, 90:10, 75:25, etc. Here, in FIG. 1, the ratio of input ports to output ports of the tap coupler 13 is 2:1, but the ratio may be set to 2:2.

The reflection mechanism 31 serves to reflect the monitor-related light TL propagated through the optical fiber 50. The reflection mechanism 31 is composed of, for example, an FBG (Fiber Bragg Grating) or reflective film, and is provided on the distal end side of the optical fiber 50 in the optical fiber probe 30. Here, when the reflection mechanism 31 is composed of a reflective film, the reflection mechanism 31 is preferably provided at the distal end of the optical fiber 50. Alternatively, when the reflection mechanism 31 is composed of an FBG, the reflection mechanism 31 is preferably provided slightly inside the distal end of the optical fiber 50.

The monitor-related light TL propagated toward the distal end side of the optical fiber 50 and reflected by the reflection mechanism 31 is further propagated back toward the proximal end side of the optical fiber 50 as the reflected light RL. Then, the reflected light RL is input to the monitor PD 12 via the connector 40 and the tap coupler 13.

Each of the optical fibers 50 is formed of, for example, a multi-mode optical fiber. This optical fiber 50 has, for example, a core diameter of 105 µm and a clad diameter of 125 µm, and is composed of a step-index type optical fiber including a coating, such as an acrylate coating film or polyimide coating film.

Here, the core diameter of the optical fiber 50 on the laser apparatus 10 side (which will be referred to as "apparatus side", hereinafter) and the core diameter of the optical fiber 50 on the optical fiber probe 30 side (which will be referred to as "probe side", hereinafter) may be different from each other. In this case, the core diameter of the optical fiber 50 on the apparatus side is preferably set smaller than the core diameter of the optical fiber 50 on the probe side.

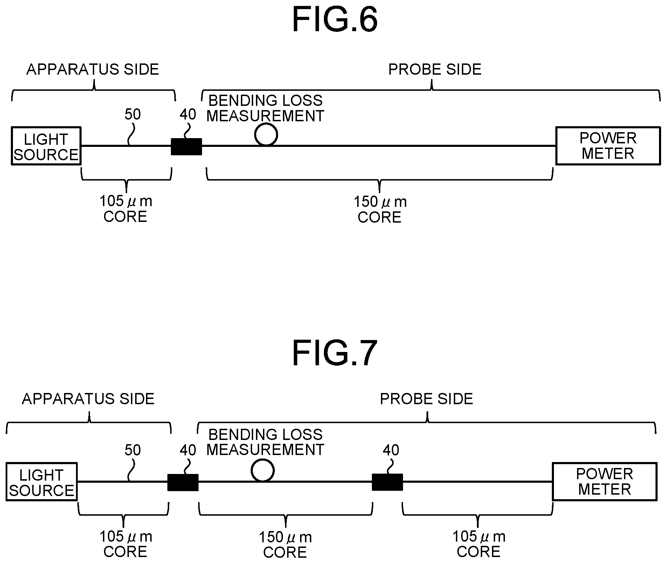

Now, FIG. 5 is a diagram for explaining an example of the bending loss characteristic of the optical fiber 50 depending on the core diameter of the optical fiber 50, which is a graph showing the relationship between the bending radius of the optical fiber 50 and the bending loss. The example 1 of FIG. 5 assumes, for example, as illustrated in FIG. 6, a system configuration which is provided with a light source on the apparatus side and a power meter on the probe side, and in which the core diameter of the optical fiber 50 on the apparatus side is 105 µm and the core diameter of the optical fiber 50 on the probe side is 150 µm. This example 1 assumes, for example, an ablation-associated line in which an ablation-related LD described later is arranged instead of the monitor-related LD 11 in FIG. 1.

The example 2 of FIG. 5 assumes, for example, as illustrated in FIG. 7, a system configuration which is provided with a light source on the apparatus side and a power meter on the probe side, and in which the core diameter of the optical fiber 50 on the apparatus side is 105 µm, the core diameter of the optical fiber 50 between two connectors 40 on the probe side is 150 µm, and the core diameter of the optical fiber 50 between the connector 40 and the power meter on the probe side is 105 µm. This example 2 assumes the reflection monitor-associated line illustrated in FIG. 1. Further, the example 3 of FIG. 5 assumes, for example, a system configuration in which the core diameter of the optical fiber 50 is 105 µm on each of the apparatus side and the probe side, in FIG. 6.

As illustrated in FIG. 5, it can be seen that, in the cases where the core diameter of the optical fiber 50 on the apparatus side and the core diameter of the optical fiber 50 on the probe side are different from each other (see the examples 1 and 2), the bending loss becomes larger and the sensitivity to the bending loss is increased, as compared with the case where the core diameters on both sides are the same as each other (see the example 3). Therefore, when the core diameter of the optical fiber 50 is set different between the apparatus side and the probe side, the apparent bending loss is increased, and makes it easier to detect the occurrence of a bend on the optical fiber 50. Here, when the core diameter of the optical fiber 50 is different between the apparatus side and the probe side, it is necessary to correct the difference in the apparent bending loss with respect to the value of the actual bending loss, by using, for example, a correlation table or the like.

The control part 60 includes an arithmetic section and a storage section, which are not illustrated. The arithmetic section serves to perform various types of arithmetic processing necessary for the control to be executed by the control part 60 and the functions to be realized by the control part 60, and is composed of, for example, a CPU (Central Processing Unit), an FPGA (Field Programmable Gate Array), or both a CPU and an FPGA. On the other hand, the storage section includes, for example, a subsection composed of a ROM (Read Only Memory) that stores various programs, data, and so forth to be used by the arithmetic section to perform arithmetic processing, and a subsection composed of a RAM (Random Access Memory) to be used as the work space when the arithmetic section performs arithmetic processing and also used for storing the result of arithmetic processing of the arithmetic section.

Further, the control part 60 includes an input section (not illustrated) that receives inputs, such as current signals from the monitor PD 12, and an output section (not illustrated) that outputs the drive current to the monitor-related LD 11 and instruction signals and various types of information to the display part 70, on the basis of the results of various types of arithmetic processing.

The display part 70 is a part that outputs various types of information to the operator of the laser apparatus 10 and displays characters and symbols to notify the outside or gives warning with an alarm or the like, in response to instruction signals from the control part 60, and is composed of, for example, a liquid crystal display.

In the optical fiber state detection system 1 configured as described above, when the control part 60 detects that the received optical power of the reflected light RL received by the monitor PD 12 is greater than 0 and lower than a predetermined threshold value, the control part 60 outputs information on the decrease in the received optical power to the outside. Specifically, the control part 60 outputs to the display part 70 such information that the received optical power has decreased. In this case, the control part 60 can determine that a bent has occurred on the optical fiber 50 in the optical fiber probe 30. The control part 60 may sound an alarm or the like by the display part 70 to notify the outside (operator) of the decrease in the received optical power, that is, the occurrence of a bend on the optical fiber 50. The predetermined threshold described above can be set by the value of the received optical power attenuated by 10% or 1 dB from the value of the received optical power under the normal condition.

In addition, when the change rate of the received optical power of the reflected light RL becomes large within a certain period of time, such as when the received optical power of the reflected light RL decreases sharply within a short period of time, for example, the control part 60 may determine that a bent has occurred on the optical fiber 50 in the optical fiber probe 30, even if the received optical power of the reflected light RL has not become lower than the predetermined threshold described above.

With the optical fiber state detection system 1 according to the first embodiment described above, the bending loss of the optical fiber 50 in operation is accurately measured, and, when the received optical power of the reflected light has decreased, information on the decrease in the received optical power can be output. Consequently, it is possible to determine the presence or absence of a bend on the optical fiber 50.

### (Second Embodiment)

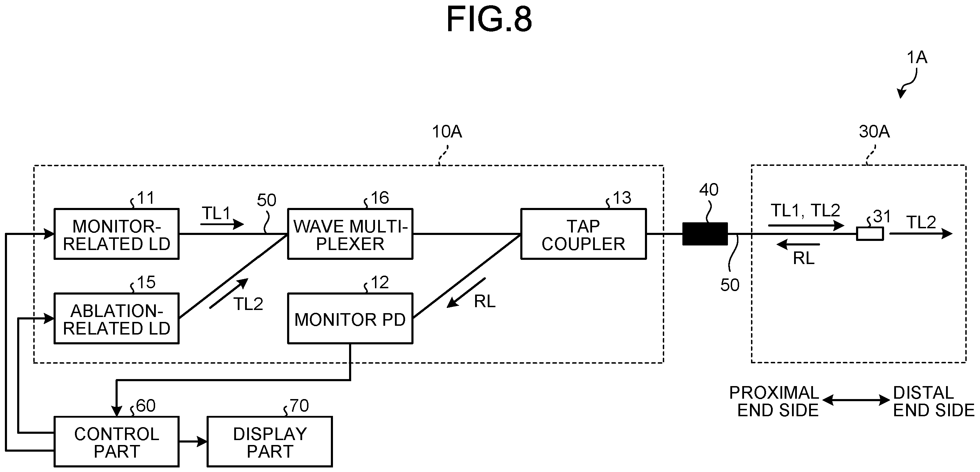

An optical fiber state detection system 1 according to this embodiment includes, as illustrated in FIG. 8, a laser apparatus 10A, an optical fiber probe 30A, a connector 40 that connects the laser apparatus 10A and the optical fiber probe 30A to each other, optical fibers 50, a control part 60, and a display part 70. Here, the configurations of the connector 40, the optical fiber 50, the control part 60, and the display part 70 in FIG. 8 are the same as those of the first embodiment described above (see FIG. 1).

The laser apparatus 10A includes a monitor-related LD 11, an ablation-related LD 15, a wave multiplexer 16, a monitor PD 12, a tap coupler 13, and optical fibers 50 that connect these components. Further, the optical fiber probe 30A is equipped with a reflection mechanism 31. Here, the optical fiber probe 30A may further include a side-face irradiation mechanism that changes the traveling direction of an ablation-related light TL2, which has been transmitted through the reflection mechanism 31, to a direction different from the traveling direction before the transmission through the reflection mechanism 31, and then radiates the light.

The monitor-related LD 11 serves as a light source (first light source) that outputs a monitor-related light TL1 for monitoring the state of an optical fiber 50. The monitor-related LD 11 is connected to the input side of the wave multiplexer 16 via an optical fiber 50. On the other hand, the monitor PD 12 is connected to the input side of the tap coupler 13 via an optical fiber 50.

The laser apparatus 10 may be provided with a plurality of monitor-related LDs 11 that output lights different from each other in wavelength. Here, for example, when two monitor-related LDs 11 are used, it is preferable to configure one monitor-related LD 11 to output a light in a wavelength bandwidth shorter than the wavelength bandwidth of the ablation-related light TL2, and configure the other monitor-related LD 11 to output a light in a wavelength bandwidth longer than the wavelength bandwidth of the ablation-related light TL2. In this way, when one monitor-related LD 11 outputs a light shorter in wavelength than the ablation-related light TL2, and the other monitor-related LD 11 outputs a light longer in wavelength than the ablation-related light TL2, the estimation accuracy of the bending loss is further improved.

The ablation-related LD 15 serves as a light source (second light source) that outputs the ablation-related light TL2. The ablation-related LD 15 is connected to the input side of the wave multiplexer 16 via an optical fiber 50. Note that, when the laser apparatus 10A is used for a laser medical treatment by a medical catheter, the ablation-related light TL2 output from the ablation-related LD 15 is a light in a wavelength bandwidth, which is the so-called "living window", that is, a light in a wavelength bandwidth of 600 nm to 1,500 nm. Here, the ablation-related light TL2 has a wavelength different from the monitor-related light TL1. Further, the output of the ablation-related LD 15 is set to 0.1 W or more, for example.

Here, the monitor-related light TL1 and the ablation-related light TL2 preferably have the same beam shape. This is because the bending loss of the optical fiber 50 is mode-dependent, so there is a case where the value changes when the bending loss is measured by a light source with a different beam shape.

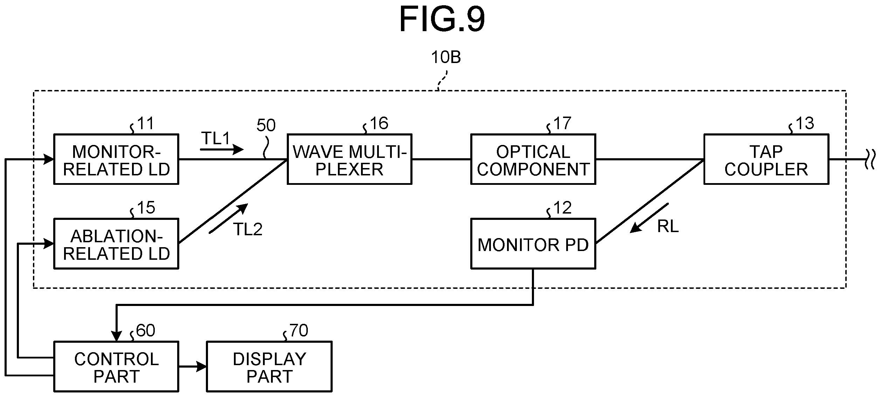

In order to make the beam shape of the monitor-related light TL1 and the beam shape of the ablation-related light TL2 the same as each other, for example, as in the laser apparatus 10B illustrated in FIG. 9, the monitor-related light TL1 and the ablation-related light TL2 are multiplexed (synthesized) on the upstream side of the laser apparatus 10B. Here, an optical component 17 illustrated in FIG. 9 is not particularly limited to a specific configuration, but may be composed of, for example, an isolator or filter, another component formed of an optical fiber, or the like. In this way, when the monitor-related light TL1 and the ablation-related light TL2 are made to have the same beam shape, the measurement error of the bending loss can be reduced.

The wave multiplexer 16 multiplexes the monitor-related light TL1 and the ablation-related light TL2. The wave multiplexer 16 is composed of, for example, a WDM (wavelength division multiplexing) coupler, combiner, tap coupler, spatial coupling optical system, or the like. The wave multiplexer 16 is connected to the input side of the tap coupler 13 via an optical fiber 50.

The reflection mechanism 31 is composed of, for example, an FBG or reflective film, and servers to reflect the monitor-related light TL1, and to transmit the ablation-related light TL2 and radiate the ablation-related light TL2 to the outside.

In an optical fiber state detection system 1A configured as described above, when the control part 60 detects that the received optical power of the reflected light RL received by the monitor PD 12 is greater than 0 and lower than a predetermined threshold value, the control part 60 outputs information on the decrease in the received optical power to the outside. Specifically, the control part 60 outputs to the display part 70 such information that the received optical power has decreased. In this case, the control part 60 can determine that a bent has occurred on the optical fiber 50 in the optical fiber probe 30A. The control part 60 may sound an alarm or the like by the display part 70 to notify the outside (operator) of the decrease in the received optical power, that is, the occurrence of a bend on the optical fiber 50. Further, the control part 60 may stop the output of the ablation-related light TL2 by shutting down the ablation-related LD 15. Here, the predetermined threshold described above can be set by the same method as in the first embodiment.

With the optical fiber state detection system 1A according to the second embodiment described above, the bending loss of the optical fiber 50 in operation is accurately measured, and, when the received optical power of the reflected light has decreased, information on the decrease in the received optical power can be output. Consequently, it is possible to determine the presence or absence of a bend on the optical fiber 50. Further, with the state detection system 1A, when a bent has occurred on the optical fiber 50, the output of the ablation-related light TL2 can be stopped by shutting down the ablation-related LD 15. Consequently, it is possible to prevent the ablation from being applied to a position not in need.

### (Third Embodiment)

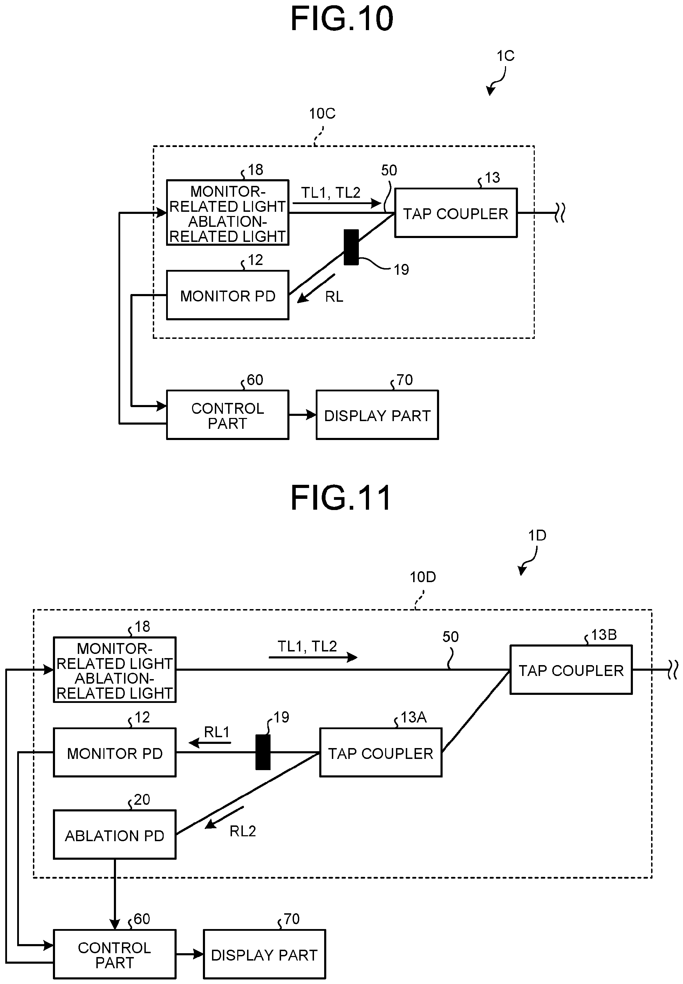

An optical fiber state detection system according to this embodiment includes a mechanism for cutting the wavelength of the ablation-related light TL2, provided on the front side of the monitor PD 12. Hereinafter, an explanation will be given of three configuration examples of this embodiment with reference to FIGS. 10 to 12. Note that, in FIGS. 10 to 12, the optical fiber probe 30A and the connector 40 are omitted from the illustration.

### <First Configuration Example>

An optical fiber state detection system 1C according to the first configuration example includes a laser apparatus 10C, which is provided with, as illustrated in FIG. 10, a light source 18, a monitor PD 12, an ablation-related light cut mechanism 19, and a tap coupler 13. Here, the configurations of the monitor PD 12 and the tap coupler 13 in FIG. 10 are the same as those of the first embodiment described above (see FIG. 1).

The light source 18 serves to output a monitor-related light TL1 and an ablation-related light TL2. The light source 18 is connected to the input side of the tap coupler 13 via an optical fiber 50. On the other hand, the monitor PD 12 is connected to the ablation-related light cut mechanism 19 via an optical fiber 50.

The ablation-related light cut mechanism 19 is arranged between the monitor PD 12 and the tap coupler 13. The ablation-related light cut mechanism 19 is composed of, for example, a filter, WDM coupler, or the like. Here, for example, when the optical fiber 50 in the optical fiber probe 30A is moved while the monitor-related light TL1 and the ablation-related light TL2 are simultaneously output from the light source 18, there is a case where part of the ablation-related light TL2 is propagated toward the monitor PD 12. In a case where the monitor PD 12 has light receiving sensitivity even at the wavelength of the ablation-related light TL2, when the monitor-related light TL1 is input to the monitor PD 12 while being overlapped with the ablation-related light TL2, the monitor PD 12 cannot properly receive only the reflected light RL derived from the monitor-related light TL1, and thus a measurement error occurs in the bending loss. In light of this, the ablation-related light cut mechanism 19 is arranged on the front side of the monitor PD 12, and thereby makes it possible to cause only a specific wavelength light (the reflected light RL derived from the monitor-related light TL1) to be input to the monitor PD 12.

### <Second Configuration Example>

An optical fiber state detection system 1D according to the second configuration example includes a laser apparatus 10D, which is provided with, as illustrated in FIG. 11, a light source 18, a monitor PD 12, an ablation PD 20, an ablation-related light cut mechanism 19, and tap couplers 13A and 13B. Here, the configurations of the light source 18, the monitor PD 12, and the ablation-related light cut mechanism 19 in FIG. 11 are the same as those of the first configuration example described above (see FIG. 10).

The light source 18 is connected to the tap coupler 13B via an optical fiber 50. On the other hand, the monitor PD 12 is connected to the ablation-related light cut mechanism 19 via an optical fiber 50. Further, the ablation PD 20 is connected to the tap coupler 13A via an optical fiber 50.

The tap coupler 13A branches a return light coming from the tap coupler 13B side into a first return light RL1 and a second return light RL2. Here, the return light includes the reflected light of the monitor-related light TL1 reflected by the reflection mechanism 31, which is not illustrated, and part of the ablation-related light TL2. The tap coupler 13A outputs the first return light RL1 to the ablation-related light cut mechanism 19 via an optical fiber 50, and outputs the second return light RL2 to the ablation PD 20. The output side of the tap coupler 13A is connected to the input side of the tap coupler 13B via an optical fiber 50.

As described above, the ablation-related light cut mechanism 19 is arranged on the front side of the monitor PD 12, and thereby makes it possible to cause only a specific wavelength light (the first return light RL1) to be input to the monitor PD 12. Here, the received optical power on the ablation PD 20 can be used to measure the power of the ablation-related light TL2.

### <Third Configuration Example>

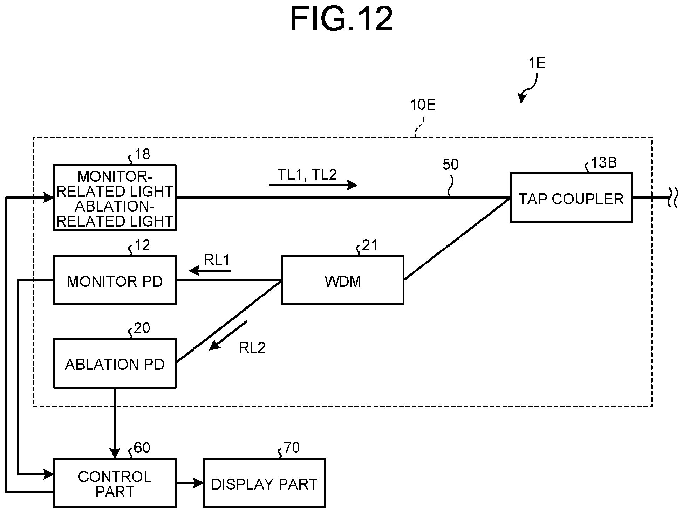

An optical fiber state detection system 1E according to the third configuration example includes a laser apparatus 10E, which is provided with, as illustrated in FIG. 12, a light source 18, a monitor PD 12, an ablation PD 20, a WDM coupler 21, and a tap coupler 13B. Here, the configurations of the light source 18, the monitor PD 12, the ablation PD 20, and the tap coupler 13B in FIG. 12 are the same as those of the second configuration example described above (see FIG. 11).

The light source 18 is connected to the tap coupler 13B via an optical fiber 50. On the other hand, the monitor PD 12 is connected to the WDM coupler 21 via an optical fiber 50. Further, the ablation PD 20 is connected to the WDM coupler 21 via an optical fiber 50.

The WDM coupler 21 demultiplexes a first return light RL1 and a second return light RL2. Then, the WDM coupler 21 outputs the first return light RL1 to the monitor PD 12, and outputs the second return light RL2 to the ablation PD 20, via the optical fibers 50. The output side of the WDM coupler 21 is connected to the input side of the tap coupler 13B via an optical fiber 50.

As described above, the WDM coupler 21 is arranged on the front side of the monitor PD 12 and the ablation PD 20, and thereby makes it possible to cause only a specific wavelength light (the first return light RL1 or second return light RL2) to be input to each of the monitor PD 12 and the ablation PD.

In the above descriptions, the optical fiber bend detection system according to each of the embodiments of the present invention has been specifically explained in the form for implementing the invention. However, the gist of the present invention is not limited to these descriptions, and should be broadly interpreted on the basis of the scope of the claims. Further, it goes without saying that various changes, modifications, and so forth based on these descriptions are also included in the gist of the present invention.

For example, in the embodiments described above, the explanations have been given on the assumption that the laser apparatus 10, 10A, 10B, 10C, 10D, or 10E is used for a medical catheter or the like, but the application of the laser apparatus 10, 10A, 10B, 10C, 10D, or 10E is not limited to the medical use.

Further, in the embodiments described above, the explanations have been given of the examples in each of which the reflection mechanism 31 is provided at the distal end of the optical fiber 50 in the optical fiber probe 30. However, the reflection mechanism 31 may be provided between (in the middle of) the distal end and the proximal end of the optical fiber 50 in the optical fiber probe 30. In this case, it is possible to determine whether or not a bent has occurred on the part of the optical fiber 50 beyond the position where the reflection mechanism 31 is provided.

### Industrial Applicability

The present invention is suitable for the application to detect a bend on an optical fiber used in an optical fiber probe for medical use.

### Reference Signs List

1, 1A, 1C, 1D, 1E state detection system
10, 10A, 10B, 10C, 10D, 10E laser apparatus
11 monitor-related LD
12 monitor PD
13, 13A, 13B tap coupler
14 combiner
15 ablation-related LD
16 wave multiplexer
17 optical component
18 light source
19 ablation-related light cut mechanism
20 ablation PD
21 WDM coupler
30, 30A optical fiber probe
31 reflection mechanism
40 connector
50 optical fiber
60 control part
70 display part

## Claims

1. An optical fiber state detection system (1) comprising:
a first light source (11) that outputs a monitor-related light for monitoring a state of an optical fiber (50);
a reflection mechanism (31) that reflects the monitor-related light propagated through the optical fiber (50);
a light receiving part (12) that receives a reflected light reflected by the reflection mechanism (31);
a tap coupler (13) provided between the reflection mechanism (31) and both the first light source (11) and the light receiving part (12) such that the first light source (11) and the light receiving part (12) are connected the tap coupler (13);
a second light source (15) that outputs an ablation-related light;
a wave multiplexer (16) that multiplexes the monitor-related light and the ablation-related light; and
a control part (60),
wherein
the first light source (11) and the second light source (15) are connected to the wave multiplexer (16),
the wave multiplexer (16) and the light receiving part (12) are connected to the tap coupler (13),
the monitor-related light and the ablation-related light are different from each other in wavelength,
the reflection mechanism (31) transmits the ablation-related light,
when the control part (60) detects that a received optical power of the reflected light is greater than 0 and lower than a predetermined threshold value, the control part (60) shuts down the second light source (15), and
the monitor-related light and the ablation-related light are same as each other in beam shape.

2. The optical fiber state detection system (1) according to claim 1, wherein a wavelength of the monitor-related light is a wavelength of 400 nm to 1,500 nm, and a wavelength of the ablation-related light is a wavelength of 600 nm to 1,500 nm.

3. The optical fiber state detection system (1) according to claim 1 or 2, wherein the monitor-related light is a flat top beam.

4. The optical fiber state detection system (11) according to any one of claims 1 to 3, wherein a mechanism (19) that cuts a wavelength of the ablation-related light is provided on a front side of the light receiving part (12).

## Patentansprüche

1. Erfassungssystem (1) für einen Zustand einer optischen Fasern, das aufweist:
eine erste Lichtquelle (11), die ein überwachungsbezogenes Licht zum Überwachen eines Zustands einer optischen Faser (50) ausgibt;
einen Reflexionsmechanismus (31), der das überwachungsbezogene Licht reflektiert, das durch den optischen Filter (50) propagiert ist;
ein lichtempfangendes Teil (12), das ein reflektiertes Licht empfängt, das durch den Reflexionsmechanismus (31) reflektiert worden ist;
einen Anzapfkoppler (13), der zwischen dem Reflexionsmechanismus (31) und beiden, der ersten Lichtquelle (11) und dem lichtempfangenden Teil (12) vorgesehen ist, so dass die erste Lichtquelle (11) und das lichtempfangende Teil (12) an den Anzapfkoppler (13) angeschlossen sind;
eine zweite Lichtquelle (15), die ein ablationsbezogenes Licht ausgibt;
einen Wellenmultiplexer (16), der das überwachungsbezogene Licht und das ablationsbezogene Licht multiplext; und
ein Steuerteil (60),
wobei
die erste Lichtquelle (11) und die zweite Lichtquelle (15) an den Wellenmultiplexer (16) angeschlossen sind,
der Wellenmultiplexer (16) und das Licht empfangende Teil (12) an den Anzapfkoppler (13) angeschlossen sind,
das überwachungsbezogene Licht und das ablationsbezogene Licht unterschiedlich voneinander bezüglich der Wellenlänge sind,
der Reflexionsmechanismus (31) das ablationsbezogene Licht überträgt,
wenn das Steuerteil (60) erfasst, das eine empfangene optische Leistung des reflektierten Lichtes größer als 0 und kleiner als ein vorbestimmter Schwellenwert ist, wobei das Steuerteil (60) die zweite Lichtquelle (15) herunterschaltet bzw. abschaltet, und
das überwachungsbezogene Licht und das ablationsbezogene Licht sind in ihrer Strahlform einander gleich.

2. Detektionssystem (1) für einen Zustand einer optischen Faser gemäß Anspruch 1, wobei eine Wellenlänge von dem überwachungsbezogenen Licht eine Wellenlänge von 400 nm bis 1,500 nm ist, und eine Wellenlänge von dem ablationsbezogenen Licht eine Wellenlänge von 600 nm bis 1,500 nm ist.

3. Erfassungssystem (1) für einen Zustand einer optischen Faser gemäß Anspruch 1 oder 2, wobei das überwachungsbezogene Licht ein flacher Spitzenstrahl ist.

4. Erfassungssystem (11) für einen Zustand einer optischen Faser gemäß irgendeinem der Ansprüche 1 bis 3, wobei ein Mechanismus (19), der eine Wellenlänge von dem ablationsbezogenen Licht schneidet bzw. abschneidet, auf einer Vorderseite von dem lichtempfangenden Teil (12) vorgesehen ist.

## Revendications

1. Système de détection de fibre optique (1), comprenant :
une première source de lumière (11) qui produit en sortie une lumière connexe à surveillance pour surveiller un état d'une fibre optique (50) ;
un mécanisme de réflexion (31) qui reflète la lumière connexe à surveillance propagée à travers la fibre optique (50) ;
une partie de réception de lumière (12) qui reçoit une lumière réfléchie, réfléchie par le mécanisme de réflexion (31) ;
un coupleur de raccordement (13) prévu entre le mécanisme de réflexion (31) et les deux de la première source de lumière (11) et de la partie de réception de lumière (12) de manière telle que la première source de lumière (11) et la partie de réception de lumière (12) sont connectées au coupleur de raccordement (13) ;
une seconde source de lumière (15) qui produit en sortie une lumière connexe à ablation ;
un multiplexeur d'onde (16) qui multiplexe la lumière connexe à surveillance et la lumière connexe à ablation ; et
une partie de commande (60), dans lequel
la première source de lumière (11) et la seconde source de lumière (15) sont connectées au multiplexeur d'onde (16),
le multiplexeur d'onde (16) et la partie de réception de lumière (12) sont connectés au coupleur de raccordement (13),
la lumière connexe à surveillance et la lumière connexe à ablation sont différentes l'une de l'autre en longueur d'onde,
le mécanisme de réflexion (31) transmet la lumière connexe à ablation,
lorsque la partie de commande (60) détecte qu'une puissance optique reçue de la lumière réfléchie est supérieure à 0 et inférieure à une valeur seuil prédéterminée, la partie de commande (60) éteint la seconde source de lumière (15), et
la lumière connexe à surveillance et la lumière connexe à ablation sont identiques l'une à l'autre en forme de faisceau.

2. Système de détection de fibre optique (1) selon la revendication 1, dans lequel une longueur d'onde de la lumière connexe à surveillance est une longueur d'onde de 400 nm à 1 500 nm, et une longueur d'onde de la lumière connexe à ablation est une longueur d'onde de 600 nm à 1 500 nm.

3. Système de détection de fibre optique (1) selon la revendication 1 ou 2, dans lequel la lumière connexe à surveillance est un faisceau à sommet plat.

4. Système de détection de fibre optique (11) selon l'une quelconque des revendications 1 à 3, dans lequel un mécanisme (19) qui coupe une longueur d'onde de la lumière connexe à ablation est prévu sur un côté avant de la partie de réception de lumière (12).
